# EUROPÄISCHE PATENTSCHRIFT

(11) **EP 1 389 450 B1**
(45) Veröffentlichungstag und Bekanntmachung des Hinweises auf die Patenterteilung: **23.03.2011**
(21) Anmeldenummer: 03001667.9
(22) Anmeldetag: 24.01.2003
(51) Int. Cl.: A61F 2/00

(54) **Hernienimplantat**
Hernia implant
Implant herniaire

(30) Priorität: 16.08.2002 DE 10237531
(43) Veröffentlichungstag der Anmeldung: 18.02.2004
(73) Patentinhaber: Tutogen Medical GmbH, 91077 Neunkirchen am Brand (DE)
(72) Erfinder: Zätsch, Steffen, 92342 Freystadt (DE); Schöpf, Christoph, 91096 Möhrendorf (DE)
(74) Vertreter: Manitz, Finsterwald & Partner GbR

(56) Entgegenhaltungen:
- WO-A1-02/22047
- WO-A1-99/16381
- FR-A1- 2 808 185
- US-A- 5 955 110
- US-A1- 2002 042 658
- US-A1- 2002 095 218

## Beschreibung

Die vorliegende Erfindung betrifft ein Hernienimplantat nach dem Oberbegriff des Anspruchs 1 zur Verwendung im Bereich der Hernienchirurgie (vgl. WO-A-99/16381).

Eine Hernie ist die Ausstülpung des Bauchfells durch eine Lücke in der Bauchwand, auch Bruch genannt. Je nach Lokalisation der Bruchpforte unterscheidet man Leistenhernien, Nabelhernien, Schenkelhernien und Narbenhernien.

Neben allgemeiner Bindegewebsschwäche begünstigen vor allem die Durchtrittsstellen von Nabelschnur und Samenstrang eine Hernienbildung. Bei Narbenhernien ist die sehr lange Heilung des Bauchschnittes eine Ursache.

Nach gegenwärtigem Stand der medizinischen Erkenntnisse werden zur Deckung der Bruchlücke bzw. zur Verstärkung der Bauchwand bei Hernienoperationen nahezu ausschließlich synthetische Materialien eingesetzt. Im Wesentlichen werden hierbei nicht resorbierbare Netze und Membranen verwendet. Resorbierbare Netze werden aufgrund des schnellen Reißkraftverlustes nur noch selten eingesetzt.

Bekannte resorbierbare Netze bestehen aus Polypropylen, Polyethylenterephtalat oder Polytetrafluoräthylen. Aufgrund von Gewebereaktionen des Körpers werden derartige Kunststoffnetze bindegewebartig abgekapselt. Hierbei ist die Dicke der Kapsel direkt proportional zur Menge an eingebrachtem Fremdmaterial. Die entstandenen Kapseln schrumpfen mit der Zeit, weshalb die Netze die Ränder der Bruchlücke mindestens etwa 5 bis 8 cm überlappen müssen. Aufgrund der hier erforderlichen ausgedehnten Präparation ist das Risiko für Hämatome und Serome ausgeprägt. Zudem kann es durch unterschiedliche Druckverteilungen zur Wanderung der Netze durch die Bauchwand kommen, weshalb derartige Netze fixiert werden müssen.

Grundsätzlich sind nicht resorbierbare Netze anfällig für Implantatinfektionen, was insbesondere für engmaschige Netze gilt. Aufgrund von Netzinfektionen ist häufig eine Explantation erforderlich.

Aufgrund der oben beschriebenen Probleme liegt bei der Verwendung von resorbierbaren und nicht resorbierbaren Netzen die Rezidivrate bei etwa 5 bis 20 %.

Es ist die Aufgabe der vorliegenden Erfindung, ein Hernienimplantat zu schaffen, das die oben genannten Probleme beseitigt.

Die Lösung dieser Aufgabe erfolgt durch die Merkmale des Anspruchs 1.

Das erfindungsgemäße Hernienimplantat wird beispielsweise aus bovinem Perikard, humanem Perikard oder aus Faszia lata gewonnen und konserviert sowie sterilisiert. Die Drainageöffnungen sind im Gegensatz zu herkömmlichen Herniennetzen, bei denen die Netzform zur Reduzierung der Menge an eingebrachtem Fremdmaterial dient, zum Schutz des Implantates selbst vorgesehen. Die Erfinder haben nämlich erkannt, dass postoperativ entstehendes Wundexudat, so genanntes Serom, eine erhöhte Anzahl an Makrophagen enthält und diese einen unerwünschten Abbau der Kollagenstruktur bewirken. Um zu verhindern, dass das Implantat schneller abgebaut als in körpereigenes Bindegewebe umgebaut wird, ist erfindungsgemäß eine Ableitung der Seromflüssigkeit aus dem Zwischenraum zwischen Muskulatur und Implantat gewährleistet, indem die voneinander beabstandeten Drainageöffnungen vorgesehen sind.

Das erfindungsgemäße Hernienimplantat kann entweder auf Stoß eingenäht oder mit einer nur geringen Überlappung von etwa 1 cm aufgelegt werden, da es nicht schrumpft. Dies erlaubt - verglichen zu herkömmlichen Implantaten - eine Verwendung von kleineren Implantaten, wodurch weniger Fremdmaterial in den Körper implantiert wird und zusätzlich erhebliche Kosten eingespart werden. Das Implantat ist ferner weitestgehend infektionsresistent und es wandert nicht innerhalb des Körpers, da es die gleiche Biomechanik wie die Bauchwand selbst aufweist. Das Implantat kann in unmittelbarem Kontakt mit den Eingeweiden implantiert werden, ohne Reizungen oder Verwachsungen zu verursachen. Der Umbau des Implantates in Bindegewebe erfolgt im Rahmen der Wundheilung, wodurch ein nur geringes Risiko von Rezidiven besteht.

Das ausschließlich aus einer Kollagenmembran bestehende Implantat ist sehr weich und geschmeidig. Es gibt keine scharfen oder harten Kanten, die Irritationen auslösen könnten. Ein weiterer Vorteil ist, dass das Implantat im Verlauf des kontinuierlich ablaufenden Umbauprozesses in vitales, körpereigenes Bindegewebe umgebaut wird.

Die Zielindikationen des erfindungsgemäßen Hernienimplantats sind vielfältig. So kann dieses bei jüngeren Patienten und Sportlern eingesetzt werden, bei denen ein einfaches Nähen als Verschluss aufgrund der körperlichen Belastung nicht angezeigt ist.

Ein weiteres Anwendungsgebiet liegt im Ersatz infizierter Netze. Da bei einem Einsatz von Kunststoffnetzen die Gefahr einer Infektion aufgrund der spezifischen Eigenschaften dieser Netze hoch ist, kann es teilweise noch nach Jahren zu Komplikationen kommen. Diese Netze müssen dabei entfernt und durch andere Materialien ersetzt werden. Da ein erneuter Einsatz eines Kunststoffnetzes im infizierten Bereich kontraindiziert wäre, lässt sich hier auf vorteilhafte Weise das erfindungsgemäße Hernienimplantat verwenden.

Ein weiterer vorteilhafter Anwendungsbereich liegt auf dem Gebiet der Narbenhernien, die als Ergebnis von chirurgischen Eingriffen im Bauchraum entstehen können, bei denen die Bauchwand im Rahmen der Eröffnung durchtrennt werden muss. Da die Heilung der Bauchwand einen langen Zeitraum beansprucht, führt die dadurch entstandene Schwäche zur Hernie. Bei der Therapie dieser Hernien wird ein Implantat benötigt, das der Bauchwand sofort nach der Implantation Halt gibt, was bei dem erfindungsgemäßen Hernienimplantat der Fall ist.

Da die Drainageöffnungen des Implantates gleichmäßig voneinander beabstandet und netzgitterartig verteilt sind, ist sichergestellt, dass das Serom gleichmäßig und ganzflächig abfließen kann, wodurch die Kollagenmembran ganzflächig geschützt wird.

Die Drainageöffnungen weisen einen gegenseitigen Abstand von etwa 10 mm auf und sind als Rundperforationen ausgebildet.

Die Drainageöffnungen sind entlang eines Quadratgitters über die gesamte Kollagenmembran verteilt, wobei ein Randbereich vorgesehen ist, der nicht mit Drainageöffnungen versehen ist.

Ferner ist es vorteilhaft für den Einheilungsprozess, wenn das Hernienimplantat ausschließlich aus der Kollagenmembran besteht, die vorzugsweise einschichtig ausgebildet ist.

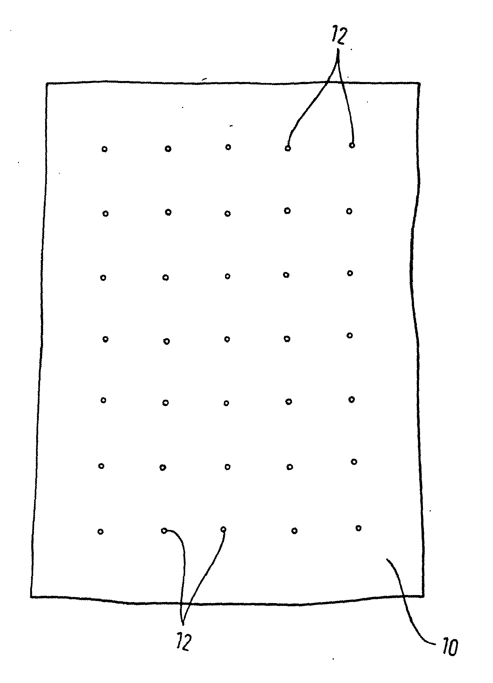

Nachfolgend wird rein beispielhaft eine Ausführungsform der Erfindung anhand der beigefügten Zeichnung beschrieben.

Die einzige Figur zeigt eine perspektivische Ansicht eines Hernienimplantats.

Das in der Figur dargestellte Hernienimplantat besteht aus einer einschichtigen Kollagenmembran 10, die aus biologischem Ausgangsmaterial gewonnen ist, beispielsweise auf bovinem Perikard. Die Kollagenmembran 10 ist rechteckig ausgebildet und weist Abmessungen von etwa 4 x 5 cm auf. Weitere denkbare Größen sind 6 x 8 cm, 8 x 12 cm und 12 x 16 cm. Andere Größen und Formen, z.B. runde oder mit Schlitzen versehene Formen können ebenfalls vorteilhaft sein.

Die in der Figur dargestellte Kollagenmembran 10 weist eine Vielzahl von Drainageöffnungen 12 auf, die entlang eines Quadratgitters angeordnet sind und die einen gegenseitigen Abstand von etwa 10 mm aufweisen. Jede Drainageöffnung 12 ist als Rundperforation mit einem Durchmesser von etwa 1 mm ausgebildet. Ein umlaufender Randbereich mit einer Breite von etwa 10 mm ist perforationsfrei ausgebildet. Bei dem dargestellten Ausführungsbeispiel sind 35 Drainageöffnungen vorgesehen.

## Patentansprüche

1. Hernienimplantat, bestehend aus einer aus biologischem Ausgangsmaterial gewonnenen Kollagenmembran (10), die mit mehreren voneinander beabstandeten Drainageöffnungen (12) versehen ist, die als Rundperforationen ausgebildet sind, wobei ein umlaufender Randbereich vorgesehen ist, der nicht mit Drainageöffnungen (12) versehen ist,
**dadurch gekennzeichnet, dass**
der Randbereich etwa 10 mm breit ist,
alle Drainageöffnungen einen Durchmesser von etwa 1 mm aufweisen,
die Drainageöffnungen (12) entlang eines Quadratgitters über die gesamte Kollagenmembran (10) verteilt sind, und
die Drainageöffnungen gleichmäßig voneinander beabstandet sind und einen gegenseitigen Abstand von etwa 10 mm aufweisen.

2. Hernienimplantat nach Anspruch 1,
**dadurch gekennzeichnet, dass**
die Kollagenmembran aus bovinem oder humanem Perikard oder Faszia lata gewonnen ist.

3. Hernienimplantat nach Anspruch 1,
**dadurch gekennzeichnet, dass**
dieses ausschließlich aus der Kollagenmembran besteht.

4. Hernienimplantat nach Anspruch 3,
**dadurch gekennzeichnet, dass**
die Kollagenmembran einschichtig ausgebildet ist.

## Claims

1. A hernia implant composed of a collagen membrane (10) obtained from a biological starting material which is provided with a plurality of drainage openings (12) separated from one another and formed as round perforations, wherein a circumferential boundary region is provided which is not provided with drainage openings (12) **characterized in that**
the boundary region is approximately 10 mm wide,
all drainage openings have a diameter of approximately 1 mm,
the drainage openings (12) are distributed along a square grid over the total collagen membrane (10), and
the drainage openings (12) are uniformly separated from one another and have a respective spacing of approximately 10 mm.

2. A hernia implant in accordance with claim 1
**characterized in that**
the collagen membrane is obtained from bovine pericardium or human pericardium or fascia lata.

3. A hernia implant in accordance with claim 1
**characterized in that**
this exclusively consists of the collagen membrane.

4. A hernia implant in accordance with claim 3
**characterized in that**
the collagen material is formed as a single layer.

## Revendications

1. Implant herniaire, constitué d'une membrane de collagène (10) obtenue à partir d'un matériau de départ biologique, laquelle est pourvue de plusieurs ouvertures de drainage (12) écartées les unes des autres, qui sont réalisées sous forme de perforations rondes, et dans laquelle est prévue une zone de bordure périphérique qui n'est pas pourvue d'ouvertures de drainage (12),
**caractérisé en ce que**
la zone de bordure a environ 10 mm de largeur,
toutes les ouvertures de drainage ont un diamètre d'environ 1 mm,
les ouvertures de drainage (12) sont réparties sur la totalité de la membrane de collagène (10) le long d'une grille à base carrée, et
les ouvertures de drainage sont écartées régulièrement les unes des autres et présentent une distance réciproque d'environ 10 mm.

2. Implant herniaire selon la revendication 1,
**caractérisé en ce que** la membrane de collagène est obtenue à partir de péricarde ou de fascia lata (tissu conjonctif) bovin ou humain.

3. Implant herniaire selon la revendication 1,
**caractérisé en ce que** celui-ci est constitué exclusivement par la membrane de collagène.

4. Implant herniaire selon la revendication 3,
**caractérisé en ce que** la membrane de collagène est réalisée avec une seule couche.
